# EUROPÄISCHE PATENTSCHRIFT

(11) **EP 4 326 530 B1**
(45) Veröffentlichungstag und Bekanntmachung des Hinweises auf die Patenterteilung: **04.11.2026**
(21) Anmeldenummer: 22722444.1
(22) Anmeldetag: 12.04.2022
(51) Int. Cl.: B29C 64/124, A61F 2/02, B29C 64/35, B29C 64/277, B29C 64/386, B29C 71/00, B33Y 10/00, B33Y 40/20, B33Y 50/00, B33Y 70/00, B33Y 70/10, B33Y 80/00

(54) **VERFAHREN ZUR HERSTELLUNG EINES MONOLITHISCHEN BAUTEILS**
PROCESS FOR MANUFACTURING A MONOLITHIC PART
PROCÉDÉ DE FABRICATION D'UNE PIÈCE MONOLITHIQUE

(30) Priorität: 21.04.2021 DE 102021110029
(43) Veröffentlichungstag der Anmeldung: 28.02.2024
(73) Patentinhaber: Anwerina AG, 8274 Tägerwilen (CH)
(72) Erfinder: SCHEITHAUER, Uwe, 01309 Dresden (DE); KARL, Christoph, 8274 Tägerwilen (CH)
(74) Vertreter: Sommer, Andrea
(86) Internationale Anmeldenummer: PCT/EP2022/059684
(87) Internationale Veröffentlichungsnummer: WO 2022/223359

(56) Entgegenhaltungen:
- EP-A1- 3 101 477
- EP-A1- 3 338 814
- JP-A- H07 171 898
- US-A1- 2018 345 580

## Beschreibung

Die vorliegende Erfindung bezieht sich auf ein Verfahren zum Ausbilden eines monolithischen Bauteils und insbesondere auf ein Verfahren zum Ausbilden eines Gelenkimplantats zur Gewebeneubildung am Gelenk oder eines technischen Funktionskörpers unter Verwendung einer additiven lithographiebasierten Fertigungstechnik.

Aus der Druckschrift WO 2010/045950 A1 ist ein Verfahren zum Ausbilden eines monolithischen Bauteils unter Verwendung einer lithographiebasierten Fertigungstechnik bekannt, wobei ein lichtpolymerisierbares Material in einer Wanne mit transparentem Wannenboden vorgesehen ist, eine Plattform in das lichtpolymerisierbare Material bis zu einem vorbestimmten Abstand zum Wannenboden eingetaucht wird, ein selektives Belichten durch den Wannenboden durchgeführt wird, wodurch ein an der Plattform anhaftender polymerisierter Schichtkörper mit einer dem Abstand entsprechenden Höhe erzeugt wird, die Plattform mit dem polymerisierten Schichtkörper zur Bildung eines nachfolgenden Schichtkörpers vertikal verschoben wird, und durch Wiederholen dieser Schichtherstellungs-Schritte schichtweise das monolitische Bauteil in der gewünschten Form aufgebaut wird.

Aus der Druckschrift US 2018/345580 A1 ist ein Verfahren zur Herstellung eines monolithischen Bauteils unter Verwendung einer additiven lithographiebasierten Fertigungstechnik bekannt, wobei in gezielt ausgewählten Bereichen unterschiedliche Oberflächentopologien erzeugt werden. Hierbei wird zur Realisierung von Mikro-Strukturen auf Makro-Objekten eine dynamische Blende mit unterschiedlichen Öffnungsgrößen oder - formen verwendet. Die Mikro-Strukturen bestimmen hierbei die funktionalen Oberflächeneigenschaften des Makro-Objekts.

Aus der Druckschrift EP 3 338 814 A1 ist ein weiteres herkömmliches Verfahren zur Herstellung eines monolithischen Bauteils unter Verwendung einer additiven lithographiebasierten Fertigungstechnik bekannt, wobei als Bauteil ein Implantat in einer CAD-Umgebung entworfen und anschließend in einer Bauraum-Umgebung additiv hergestellt wird.

Die Druckschrift JP H07 171898 A bezieht sich lediglich auf ein herkömmliches Druck- bzw. Kopiergerät, welches eine Fixiereinheit mit einer Fixierwalze und eine Trenneinheit zum Trennen eines Papiers von der Fixierwalze verwendet.

Schließlich ist aus der Druckschrift EP 3 101 477 A1 ein herkömmliches 3D-Druckverfahren bekannt, wobei ein Belichtungs-System mit einzeln ansteuerbaren Mikrospiegeln beim Belichten des verwendeten lichtsensitiven Materials zum Einsatz kommt.

In vielen technischen Bereichen ist es notwendig Materialien herzustellen, die an unterschiedlichen Oberflächenbereichen unterschiedliche Oberflächeneigenschaften wie z.B. ein Benetzungsverhalten aufweisen.

Unter Benetzungsverhalten wird nachstehend das Verhalten von Flüssigkeiten bei Kontakt mit einer Festkörperoberfläche verstanden. Die Benetzbarkeit ist hierbei die zugehörige Eigenschaft der Festkörperoberfläche für eine bestimmte Flüssigkeit und wird regelmäßig über den sogenannten Kontaktwinkel eines Flüssigkeitstropfens an der Oberfläche des Festkörpers definiert. Keine oder nur geringe Benetzung liegt hierbei vor, wenn der Kontaktwinkel in einem Bereich von 180° bis 90° liegt, wobei sich ein kugel- oder halbkugelförmiger Flüssigkeitstropfen ausbildet, der nur eine kleine (oder punktförmige) Auflagefläche aufweist und leicht über die Festkörperoberfläche gleitet (Abperlen). Eine partielle Benetzung liegt vor, wenn der Kontaktwinkel kleiner 90° ist, wobei ein Flüssigkeitstropfen an der Festkörperoberfläche eine runde Haube ausbildet (Abgleiten). Eine vollständige Benetzung liegt vor, wenn der Kontaktwinkel nahezu 0° ist, wobei sich ein Flüssigkeitstropfen an der Festkörperoberfläche in Form einer flachen Scheibe ausbreitet. In diesem Fall bleiben Flüssigkeitsreste auch bei Einwirken von starken Kräften (z.B. extreme Neigung der Oberfläche) an der Festkörperoberfläche haften.

Beim Spezialfall von Wasser als verwendete Flüssigkeit spricht man in diesem Zusammenhang von hydrophoben (wasserabweisenden) oder hydrophilen (wasseranziehenden) Festkörperoberflächen.

Sowohl bei technischen Funktionskörpern (Katalysatoren, Kondensatoren, Membrane ...) als auch bei in der Medizintechnik verwendeten Materialien und Körpern ist ein unterschiedliches Benetzungsverhalten regelmäßig gewünscht und muss bisher durch zeit- und kostentintensive Nachbehandlungsverfahren (mechanische/chemische Nachbehandlung und/oder chemische Beschichtung) nach Herstellung des Ausgangsmaterials realisiert werden.

Der Erfindung liegt daher die Aufgabe zugrunde, ein Verfahren zur Herstellung eines monolithischen Bauteils zu schaffen, wobei unterschiedliche Oberflächeneigenschaften, insbesondere hinsichtlich eines Benetzungsverhaltens, eines Abscheideverhaltens und/oder einer Besiedlung mit Zellen, lokal sowie auf einfache und kostengünstige Weise realisiert werden können.

Erfindungsgemäß wird diese Aufgabe durch die Merkmale des Patentanspruchs 1 gelöst.

So erfolgt das Ausbilden des monolithischen Bauteils unter Verwendung einer additiven Fertigungstechnik, wobei beispielsweise ein lichtpolymerisierbares Material in einer Wanne mit transparentem horizontalen Wannenboden vorgesehen ist, eine Plattform in das lichtpolymerisierbare Material bis zu einem vorbestimmten Abstand zum Wannenboden eingetaucht wird, ein selektives Belichten durch den Wannenboden durchgeführt wird, wodurch ein an der Plattform anhaftender polymerisierter Schichtkörper mit einer dem Abstand entsprechenden Höhe erzeugt wird, die Plattform mit dem polymerisierten Schichtkörper zur Bildung eines nachfolgenden Schichtkörpers vertikal verschoben wird, und durch Wiederholen der vorhergehenden Schichtherstellungs-Schritte schichtweise das monolitische Bauteil in der gewünschten Form aufgebaut wird. Durch Einstellung von Fertigungsparametern während dem Wiederholen der Schicht-Herstellungs-Schritte werden unterschiedliche Oberflächentopologien zur Realisierung der unterschiedlichen Oberflächeneigenschaften in ausgewählten Bereichen des monolithischen Bauteils erzeugt.

Ein unter Verwendung dieser lithographiebasierten 3D-Drucktechnik hergestelltes Bauteil kann besonders kostengünstig hergestellt werden, da eine Nachbearbeitung sowohl eines Grünkörpers als auch der fertig gesinterten Keramik damit erspart bleibt und auch an unzugänglichen Stellen ermöglicht ist.

Beispielsweise kann als Fertigungsparameter ein Belichtungsparameter, insbesondere eine Belichtungsintensität und/oder eine Belichtungszeit, beim selektiven Belichten durch den Wannenboden derart eingestellt werden, dass zusätzlich zum zu polymerisierenden Schichtkörper ein in den Kanten und/oder Ecken der Treppenstruktur verbleibendes lichtpolymerisierbares Restmaterial polymerisiert bzw. vernetzt wird.

Dies ermöglicht die gleichzeitige Ausbildung von unterschiedlichen Oberflächentopologien an gegenüberliegenden Oberflächenbereichen auf besonders einfache Weise.

Ferner kann als Fertigungsparameter ein Verdrehungswinkel zwischen horizontalen Entwurfselement-Hauptachsen und horizontalen Bauraum-Hauptachsen bzw. einer Reihen- oder Spaltenausrichtung einer Mikrospiegel-Einheit verändert werden. Hierdurch können für jeden polymerisierten Schichtkörper an den Seitenoberflächen unterschiedliche Oberflächentopologien ausgebildet werden.

Ferner kann als Fertigungsparameter ein Verkippungswinkel zwischen einer vertikalen Entwurfselement-Hauptachse und einer vertikalen Bauraum-Hauptachse bzw. einer Lotrechten des horizontalen Wannenbodens verändert werden.

Hierdurch können in vertikaler Richtung unterschiedliche Oberflächentopologien ausgebildet werden.

Ferner kann als Fertigungsparameter der Abstand zwischen Plattform bzw. letztem polymerisierten Schichtkörper und dem Wannenboden während dem Wiederholen der Schichtherstellungs-schritte verändert werden.

Hierdurch können in vertikaler Richtung weiterhin unterschiedliche Oberflächentopologien ausgebildet werden.

Ferner können als Fertigungsparameter die Abmessungen der von den Mikrospiegeln der Mikrospiegel-Einheit reflektierten Bildpunkte durch eine Abbildungseinheit während dem Wiederholen der Schichtherstellungsschritte verändert werden. Hierdurch können die Oberflächentopologien weiterhin verändert werden.

In den weiteren Unteransprüchen sind weitere vorteilhafte Ausgestaltungen der Erfindung gekennzeichnet.

Die Erfindung wird nachstehend anhand von Ausführungsbeispielen unter Bezugnahme auf die Zeichnung näher beschrieben.

Es zeigen:
Figur 1 eine vereinfachte Seitenansicht einer Vorrichtung zum schichtweisen Aufbau eines monolithischen Bauteils;
Figuren 2a und 2b vereinfachte Ansichten einer verwendeten Mikrospiegel-Einheit;
Figuren 3a und 3b vereinfachte perspektivische Ansichten zur Veranschaulichung von Verfahrensschritten gemäß einem ersten Ausführungsbeispiel, wobei unterschiedliche Oberflächentopologien aufgrund einer gezielten Veränderung einer Entwurfs-Orientierung eines Entwurfselements zu einer Bauraum-Orientierung ausgebildet wird;
Figuren 4a bis 4c vereinfachte Ansichten zur Veranschaulichung von Verfahrensschritten bei der selektiven Herstellung von unterschiedlichen Oberflächentopologien gemäß einem zweiten Ausführungsbeispiel, wobei eine Verdrehung in der x,y-Ebene durchgeführt wird;
Figuren 5 vereinfachte Ansichten eines stabförmigen Bauteils zur Veranschaulichung von Verfahrensschritten bei der selektiven Herstellung von unterschiedlichen Oberflächentopologien gemäß einem dritten Ausführungsbeispiel, wobei eine Verkippung gegenüber der z-Achse durchgeführt wird;
Figuren 6a bis 6c vereinfachte perspektivische Ansichten zur Veranschaulichung von Verfahrensschritten gemäß einem vierten Ausführungsbeispiel, wobei unterschiedliche Oberflächentopologien aufgrund einer Durchbelichtung an gegenüberliegenden Seiten gleichzeitig ausgebildet werden; und
Figur 7 eine vereinfachte perspektivische Ansicht eines mit dem erfindungsgemäßen Verfahren herstellbaren Gelenkimplantats zur Gewebeneubildung an einem Gelenk.

Die nachfolgende Beschreibung bezieht sich insbesondere auf ein Verfahren zur Herstellung eines Gelenkimplantats zur Gewebeneubildung an einem Gelenk. In gleicher Weise kann das Verfahren auch für die Herstellung von technischen Funktionsmaterialen verwendet werden, bei dem ein Benetzungsverhalten an unterschiedlichen Oberflächenbereichen durch gezielte Änderung der Oberflächentopologien eingestellt werden kann.

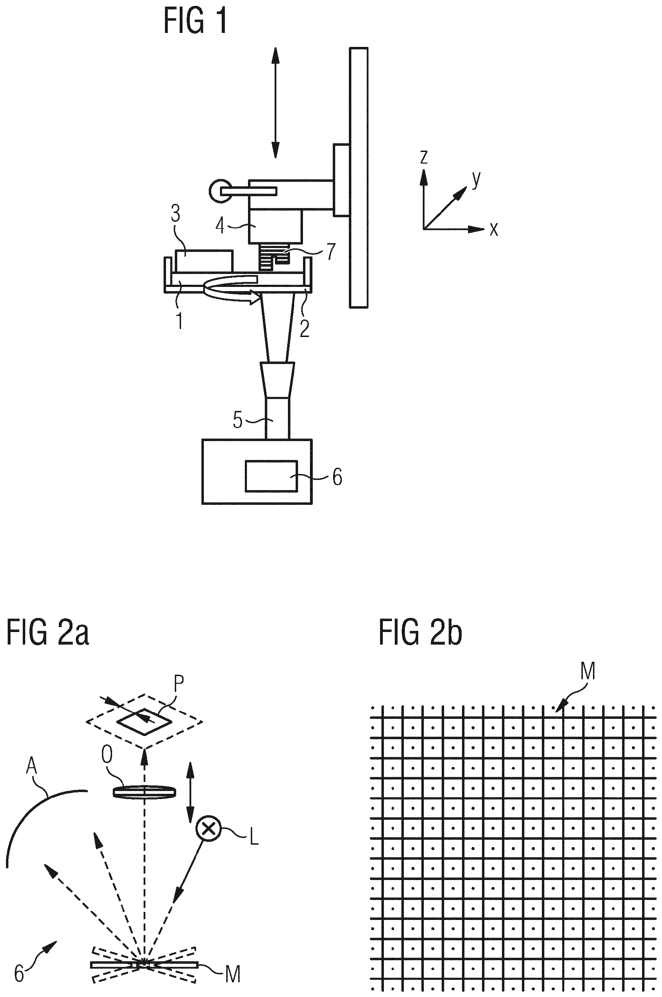

Figur 1 zeigt eine vereinfachte Seitenansicht einer Vorrichtung zum schichtweisen Aufbau eines monolithischen Bauteils, wie sie zur Realisierung des erfindungsgemäßen Verfahrens verwendet werden kann. Eine entsprechende Vorrichtung wird auch als sogenannter 3D-Drucker bezeichnet.

Ein derartiger beispielsweise von der Fa. Lithoz^{®} hergestellter 3D-Drucker basiert beispielsweise auf einer lithographiebasierten Fertigungstechnik, wobei ausgehend von einem CAD-Modell des zu fertigenden Bauteils die entsprechenden Datensätze direkt an eine nicht dargestellte Steuereinheit des 3D-Druckers übertragen werden.

Hierbei kann beispielsweise ein lichtpolymerisierbares Material 1 in eine Wanne mit transparentem horizontalen Wannenboden 2 eingebracht und beispielsweise mit einem in der Höhe einstellbaren Glättelement bzw. Rakel 3 gleichmäßig in der Wanne verteilt werden. Beispielsweise kann als lichtpolymerisierbares Material 1 eine keramische Suspension (partikelbeladene Dispersion, auch als Schlicker bezeichnet) verwendet werden. Eine in Z-Richtung (vertikal) bewegliche Plattform 4 wird hierbei in das lichtpolymerisierbare Material 1 bis zu einem vorbestimmten Abstand zum Wannenboden 2 eingetaucht und anschließend ein selektives Belichten des lichtpolymerisierbaren Materials 1 zum Ausbilden eines polymerisierten und an der Plattform anhaftenden 3D-Schichtkörpers durchgeführt. Die Höhe des polymerisierten Schichtkörpers entspricht hierbei dem vorbestimmten Abstand der Plattform 4 vom Wannenboden 2, während seine Grundfläche einer jeweiligen Belichtungsfläche entspricht. Das selektive Belichten kann folglich insbesondere als orts-selektives Belichten verstanden werden.

Gemäß Figur 1 kann die selektive Belichtung bzw. Bestrahlung des lichtpolymerisierbaren Materials 1 durch eine Belichtungs-Einheit 5 von unten durch den transparenten Wannenboden 2 durchgeführt werden. Beispielsweise kann hierbei blaues Licht der Wellenlänge von ca. 465 nm verwendet werden. Durch die selektive Belichtung kann das lichtpolymerisierbare Material 1 polymerisiert (vernetzt bzw. verfestigt) und als an der Plattform 4 anhaftender erster polymerisierter Schichtkörper eines zu erzeugenden monolithischen Bauteils bzw. Grünkörpers 7 ausgebildet werden.

Anschließend kann die Plattform 4 mit dem ersten polymerisierten Schichtkörper in Z-Richtung vertikal nach oben verschoben werden, optional lichtpolymersisierbares Material 1 nach Bedarf nachgefüllt werden, optional mit dem Glättelement 3 eingeebnet bzw. gleichmäßig verteilt werden und schließlich wieder bis zu einem vorbestimmten Abstand des zuletzt polymerisierten Schichtkörpers zum Wannenboden 2 in das lichtpolymerisierbare Material 1 eingetaucht und (orts-)selektiv belichtet werden, wodurch ein weiterer polymerisierter Schichtkörper ausgebildet wird. Durch die Wiederholung dieser Schichtherstellungsschritte entsteht schließlich das gewünschte monolithische und dreidimensionale Bauteil 7 (Grünkörper) aus einer Vielzahl von polymerisierten und aneinander haftenden Schichtkörpern, wobei eine Oberfläche des Bauteils in Z-Richtung zumindest teilweise eine treppenförmige Struktur bzw. Topologie aufweisen kann.

Gemäß Figur 1 kann die Belichtungs-Einheit 5 eine digitale Mikrospiegel-Einheit 6 aufweisen, die die selektive Belichtung des lichtpolymersisierbaren Materials 1 ermöglicht.

Die Figur 2a zeigt eine vereinfachte Ansicht einer beispielsweise verwendeten Mikrospiegel-Einheit 6 und Figur 2b zeigt eine vereinfachte Ansicht eines Ausschnitts einer zugehörigen digitalen Mikrospiegel-Vorrichtung (DMD, Digital Micromirror Device), wie sie erfindungsgemäß zur Verwendung kommen können.

Gemäß Figuren 2a und 2b kann die Mikrospiegel-Vorrichtung eine Vielzahl von in Reihen und Spalten angeordneten schwenkbaren Mikrospiegeln M aufweisen, welche von einer Lichtquelle bzw. Strahlungsquelle L bestrahlt werden können. Die Mikrospiegel M können über eine nicht dargestellte Steuereinheit derart angesteuert werden, dass sie das einfallende Licht (1. Strahl von rechts) entweder in Richtung der Plattform 4 zur Realisierung eines Bildpunktes bzw. Pixels P und zur Vernetzung des dort befindlichen lichtpolymersisierbaren Materials 1 reflektieren (2. Strahl von rechts) oder in Richtung eines Absorbers A (keine Vernetzung des im Bereich der Plattform 4 befindlichen lichtpolymersisierbaren Materials 1) ablenken (4. Strahl von rechts). In einem nicht angesteuerten (spannungslosen) Zustand kann das Licht von den schwenkbaren Mikrospiegeln M ebenfalls in Richtung des Absorbers A abgelenkt werden (3. Strahl von rechts), weshalb wiederum keine Vernetzung von lichtpolymersisierbaren Material stattfindet.

Gemäß Figur 2b kann die Mikrospiegel-Vorrichtung etwa 1000 bis 10000 Reihen und Spalten von Mikrospiegeln M aufweisen. Ferner kann ein Mikrospiegel M einen oder mehrere Pixel bzw. Bildpunkte P darstellen. Beispielsweise kann eine Mikrospiegel-Vorrichtung mit einer Auflösung von 4096 x 2100 Pixel (4K) verwendet werden. Die Mikrospiegel M können beispielsweise quadratisch mit einer Seitenlänge von ca. 10 µm bis 20 µm ausgebildet sein. Ferner kann gemäß Figur 2b ein tatsächlich am Wannenboden 2 abgebildeter Bildpunkt bzw. Pixel P eine über eine Abbildungseinheit O einstellbare bzw. veränderbare Größe aufweisen. Die optische Abbildungseinheit O ist ebenfalls über eine nicht dargestellte Steuereinheit ansteuerbar und kann eine oder mehrere Linsen aufweisen. Alternativ können die Mikrospiegel M der Mikrospiegel-Vorrichtung auch nur zwei Zustände annehmen, wobei das reflektierte Licht entweder in den Absorber A oder in die Abbildungseinheit O abgelenkt wird.

Das Verfahren zum schichtweisen Aufbau eines monolithischen Bauteils mit der Vorrichtung gemäß Figuren 1 und 2 kann beispielsweise eine Auflösung von etwa 10-100µm in X-Y-Richtung und eine Auflösung von etwa 5-100µm in Z-Richtung realisieren. Die maximalen 3D-Abmessungen für den auszubildenden Grünkörper 7 (3D-Druck) können beispielsweise 76 x 43 x 150 mm³ aufweisen.

Nach der Fertigstellung des additiv hergestellten 3D-Schichtkörpers bzw. Grünkörpers 7 kann abschließend ein Reinigen, Entbindern und Sintern durchgeführt werden, wodurch man das finale monolithische Bauteil erhalten kann.

Die in Figur 1 dargestellten Achsen X, Y und Z kennzeichnen die Hauptachsen des tatsächlichen Bauraums bzw. Fertigungsraums und werden nachfolgend auch als Bauraum-Orientierung bezeichnet.

Beispielsweise kann das lichtpolymerisierbare Material 1 ein Polymer, insbesondere PA, PEK, PEKK, UHMWPE oder PCL; ein Metall, insbesondere Ti oder Edelstahl; eine Metall-Legierung, insbesondere Ti64 oder CoCr; eine Magnesium-Legierung, insbesondere Mg-Ca, Mg-Zr oder Mg-Zn; eine Keramik, insbesondere Al2O3, ZrO2 oder Ca3(PO4)2; und/oder Si3N4 aufweisen. Dadurch kann beispielsweise ein mechanisch hochfestes Gelenkimplantat mit verbesserten Eigenschaften zur Gewebeneubildung bzw. ein technischer Funktionskörper mit verbesserten Eigenschaften hinsichtlich eines mechanischen Verschleißes, einer Sensoranwendung, einer Kondensatoranwendung, einer Katalysatoranwendung, einer Elektrophorese und/oder einer gezielten Abscheidung von Materialien realisiert werden. Der Grünkörper kann entweder als partikelgefülltes Polymerbauteil eingesetzt werden oder nach einer nachfolgenden Prozessierung als metallisches/keramisches Bauteil vorliegen.

Grundsätzlich können auch weitere medizinisch zugelassene, bioinerte und biokompatible, sowie 3D-Druck-fähige Materialien für das lichtpolymerisierbare Material 1 insbesondere zur Herstellung des Gelenkimplantats verwendet werden.

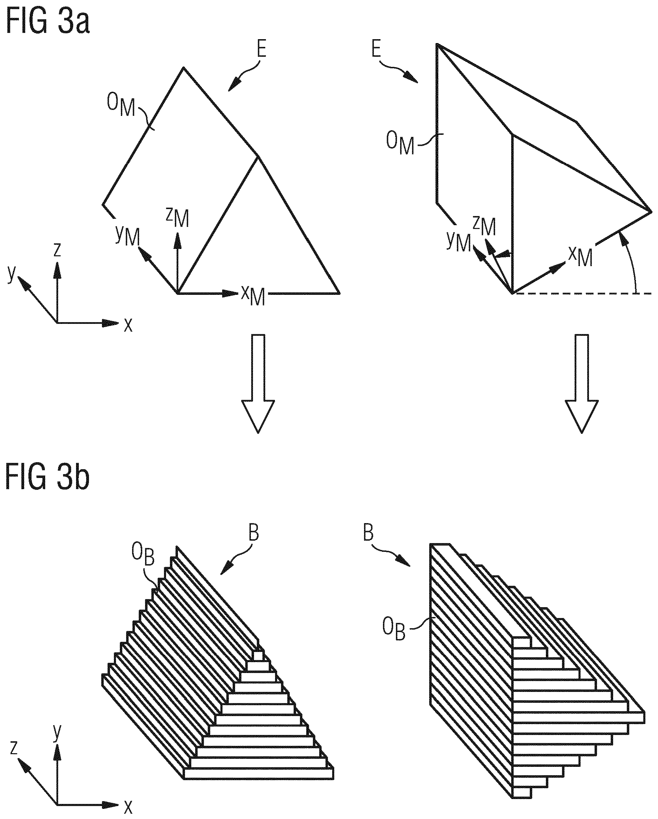

Figuren 3a und 3b zeigen vereinfachte perspektivische Ansichten zur Veranschaulichung von Verfahrensschritten gemäß einem ersten Ausführungsbeispiel, wobei unterschiedliche Oberflächentopologien aufgrund einer gezielten Veränderung einer Entwurfs-Orientierung eines Entwurfselements zu einer Bauraum-Orientierung ausgebildet werden.

In Figur 3a sind beispielhaft identische Entwurfselemente E mit unterschiedlicher Entwurfs-Orientierung (X_{M}, Y_{M}, Z_{M}) dargestellt, wie sie in einer Entwurfsebene bzw. Designebene (CAD oder CAM) durch eine Drehung erzeugt werden können. Eine Vielzahl derartiger Entwurfselemente E kann ein (nicht dargestelltes) Entwurfs-Bauteil bilden.

Figur 3b zeigt zugehörige tatsächliche Bauteilelemente B, wie sie durch eine additive Fertigungstechnik in einem Bauraum (X, Y, Z) herstellbar sind. Wie in Figur 3b dargestellt ist, ergeben sich aufgrund der additiven Fertigungstechnik (schichtweiser Aufbau) für eine zu betrachtende Entwurfsoberfläche O_{M} des linken Entwurfselements E stufenförmige Oberflächentopologien für die entsprechende Bauteiloberfläche O_{B} des linken Bauteilelements B.

Erfindungsgemäß kann nunmehr bereits beim Entwurf der jeweiligen Entwurfselemente E eine Entwurfs-Orientierung (X_{M}, Y_{M}, Z_{M}) derart gezielt verändert werden, dass für die zu betrachtende Entwurfsoberfläche O_{M} des Entwurfselements E eine unterschiedliche Oberflächentopologie und damit Oberflächeneigenschaft realisiert wird. Genauer gesagt kann beispielsweise durch die in Figur 3a für das rechte Entwurfselement E dargestellte gezielte Veränderung der Entwurfs-Orientierung (X_{M}, Y_{M}, Z_{M}) gegenüber einer Bauraum-Orientierung (X, Y, Z) in einem tatsächlichen Bauraum nunmehr eine glatte Bauteiloberfläche O_{B} erzeugt werden. Somit können in gezielt ausgewählten Bereichen eines zur erzeugenden Bauteils insbesondere hinsichtlich eines Benetzungsverhaltens, eines Abscheideverhaltens und/oder einer Besiedlung mit Zellen lokal unterschiedliche Oberflächeneigenschaften realisiert werden.

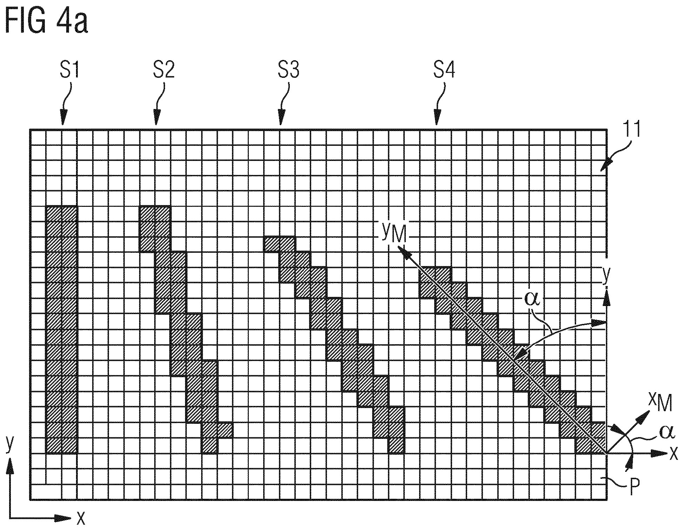

Die Figuren 4a bis 4c zeigen vereinfachte Ansichten zur Veranschaulichung von Verfahrensschritten bei der selektiven Herstellung von unterschiedlichen Oberflächentopologien gemäß einem zweiten Ausführungsbeispiel, wobei eine Verdrehung zwischen den horizontalen Bauraum-Hauptachsen bzw. projezierten Mikrospiegel-Hauptachsen X, Y gemäß Figuren 2a und 2b zu den horizontalen Entwurfselement-Hauptachsen X_{M}, Y_{M} durchgeführt wird.

Figur 4a zeigt eine vereinfachte Draufsicht eines durch die Mikrospiegeleinheit 6 auf den Wannenboden 2 projezierbaren Bildpunktfeldes, welches die horizontalen Bauraum-Hauptachsen bzw. Mikrospiegel-Hauptachsen X, Y darstellt. Üblicherweise sind die Entwurfselement-Hauptachsen X_{M}, Y_{M} des herzustellenden Bauteils parallel und nicht veränderbar zu diesen Mikrospiegel-Hauptachsen X, Y ausgerichtet (α = 0°), wodurch beispielsweise ein Schichtkörper S1 mit geraden (glatten) Seitenflächen ausgebildet werden kann. Durch Verdrehung der Entwurfselement-Hauptachsen X_{M}, Y_{M} gegenüber den Mikrospiegel-Hauptachsen X,Y um einen Verdrehungswinkel α können nunmehr unterschiedliche Oberflächentopologien an den Seitenflächen der ausgebildeten Schichtkörper S2 bis S4, wie in Figur 4a dargestellt, ausgebildet werden.

Gemäß Figur 4a kann demzufolge bei einem Verdrehungswinkel zwischen den Mikrospiegel-Hauptachsen X_{M}, Y_{M} (horizontale Bauraum-Hauptachsen) gegenüber den Entwurfselement-Hauptachsen X,Y von α = 45° eine Oberflächentopologie an den Seitenflächen für einen Schichtkörper S4 ausgebildet werden, die im Wesentlichen durch die Abmessungen der auf den Wannenboden 2 projezierten Bildpunkte bzw. Pixel P definiert ist.

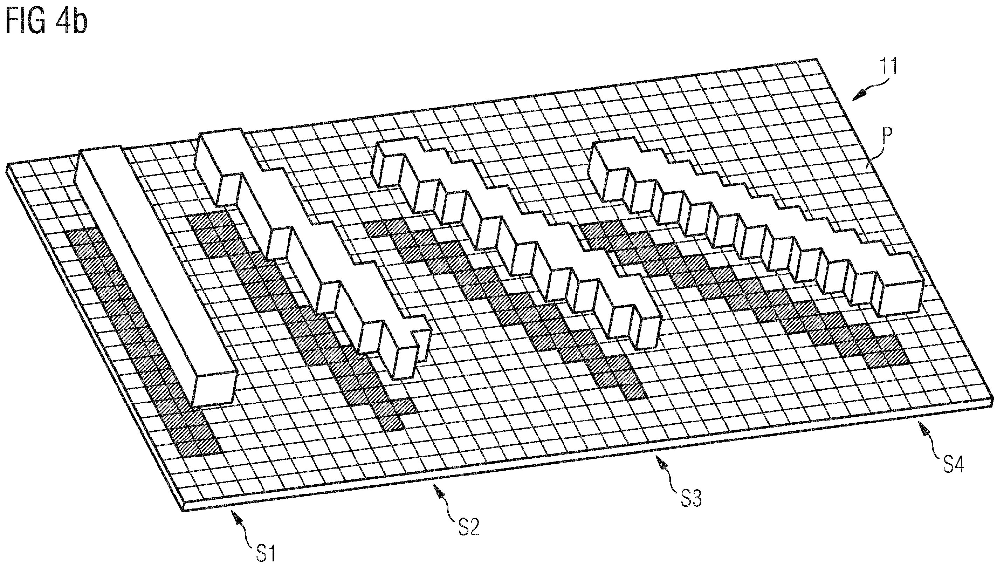

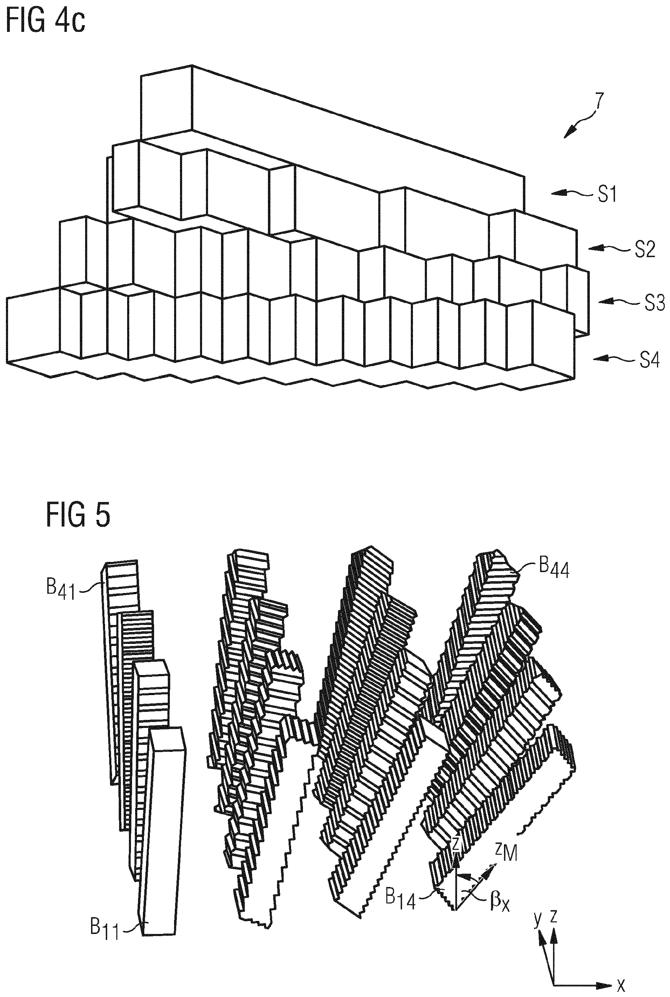

Die Figur 4b zeigt eine zugehörige perspektivische Ansicht der in Figur 4a dargestellten Draufsicht. Wie aus Figur 4c ersichtlich ist, kann bei einem aufeinanderfolgenden Schichtaufbau der Schichtkörper S1 bis S4 unter Änderung des Verdrehungswinkels α von 0° bis 45° ein 3D-Körper 7 hergestellt werden, der in seinem oberen Bereich (Schichtkörper S1) glatte Seitenoberflächen aufweist, während sein unterster Bereich (Schichtkörper S4) eine sehr raue (treppenförmige) Seitenoberfläche aufweist.

Somit kann gemäß diesem zweiten Ausführungsbeispiel durch Änderung eines Verdrehungswinkels α zwischen den horizontalen Entwurfselement-Hauptachsen X_{M}, Y_{M} und einer projezierten Reihen-/Spaltenausrichtung X,Y der Mikrospiegel-Einheit 6 während dem Wiederholen der Schichtherstellungsschritte eine Oberflächentopologie des monolithischen Bauteils 7 gezielt verändert werden, wodurch beispielsweise ein unterschiedliches Benetzungsverhalten in unterschiedlichen lokalen Bereichen realisiert werden kann.

Die Figur 5 zeigt vereinfachte Ansichten von resultierenden 3D-Schichtkörpern zur Veranschaulichung von Verfahrensschritten bei der selektiven Herstellung von unterschiedlichen Oberflächentopologien gemäß einem dritten Ausführungsbeispiel, wobei eine Verkippung zwischen einer vertikalen Entwurfselement-Hauptachse Z_{M} und einer vertikalen Bauraum-Hauptachse Z bzw. der Lotrechten des Wannenbodens 2 durchgeführt wird.

Gemäß Figur 5 bezeichnen X,Y die horizontalen Hauptachsen eines horizontalen Wannenbodens 2 und Z eine Lotrechte bzw. Senkrechte zu diesem horizontalen Wannenboden 2. Mit Z_{M} ist eine vertikale Entwurfselement-Hauptachse bezeichnet, entlang der die jeweiligen Schichtkörper wie vorstehend beschrieben ausgebildet werden.

Die Figur 5 zeigt als Bauteil einen Stab mit 16 verschiedenen Orientierungen zur Plattform bzw. zur vertikalen Hauptachse des Bauraums. Die vertikale Hauptachse Z_{M} des Entwurfs-Bauteils bzw. -Stabs B₁₁ ist parallel zur Lotrechten Z des Wannenbodens. Die Stäbe B₁₂ bis B₁₄ neigen sich in der XZ-Ebene, während sich die Stäbe B₂₁ bis B₄₁ in der YZ-Ebene neigen. Die weiteren in Figur 5a dargestellten Stäbe B₂₂ bis B₄₄ neigen sich sowohl in die XZ-Ebene als auch in die YZ-Ebene.

Die Entwurfselement-Hauptachse Z_{M} kann hinsichtlich der horizontalen Hauptachsen des Wannenbodens in X-Richtung um einen Verkippungswinkel βₓ und in Y-Richtung um einen Verkippungswinkel β_{y} gekippt werden. Ferner kann eine Verkippung sowohl in X- als auch in Y-Richtung stattfinden (βₓ, _{y}).

Die Figur 5 zeigt ein monolithisches Bauteil B₁₁, welches mit einem Verkippungswinkel von βₓ = 0 und β_{y} = 0 ausgebildet worden ist, weshalb an den vier Seitenfläche des Bauteils keinerlei treppenförmige Oberflächentopologien zu erkennen sind.

Gemäß Figur 5 sind in einer ersten Zeile drei weitere monolithische Bauteile dargestellt, wobei ein Verkippungswinkel βₓ in X-Richtung jeweils vergrößert ist. Aufgrund des schichtweisen Aufbaus und der Verkippung in X-Richtung ergibt sich bei einem Verkippungswinkel von βₓ = 45° das in Figur 5a mit B₁₄ bezeichnete monolithische Bauteil, welches an seinen zwei in X-Richtung geneigten bzw. verkippten Seitenflächen eine stufenförmige Oberflächentopologie aufweist, während die in y-Richtung nicht geneigten bzw. verkippten Seitenflächen eine glatte Oberfläche aufweisen.

In ähnlicher Weise sind gemäß Figur 5 in einer ersten Spalte drei weitere monolithische Bauteile dargestellt, wobei ein Verkippungswinkel β_{y} in Y-Richtung jeweils vergrößert ist. Aufgrund des schichtweisen Aufbaus und der Verkippung in Y-Richtung ergibt sich wiederum bei einem Verkippungswinkel von β_{y} = 45° das in Figur 5 mit B₄₁ bezeichnete monolithische Bauteil, welches an seinen beiden in Y-Richtung geneigten bzw. verkippten Seitenflächen eine stufenförmige Oberflächentopologie aufweist, während die beiden in X-Richtung nicht geneigten bzw. verkippten Seitenflächen eine glatte Oberfläche aufweisen.

In ähnlicher Weise sind gemäß Figur 5 in weiteren Reihen und Spalten weitere monolithische Bauteile dargestellt, wobei jeweils ein Verkippungswinkel βₓ in X-Richtung und β_{y} in Y-Richtung vergrößert ist. Aufgrund des schichtweisen Aufbaus und der Verkippung in X- und Y-Richtung ergibt sich beispielsweise bei einem Verkippungswinkel von βₓ = 45° und β_{y} = 45° das in Figur 5 mit B₄₄ bezeichnete monolithische Bauteil, welches an seinen vier in X- und Y-Richtung geneigten bzw. verkippten Seitenflächen jeweils stufenförmige Oberflächentopologien aufweist.

Die stufen- bzw. treppenförmigen Oberflächentopologien können somit auch durch eine Verkippung der vertikalen Entwurfselement-Hauptachse Z_{M} zu einer vertikalen Bauraum-Hauptachse Z bzw. der Lotrechten des horizontalen Wannenbodens 2 gezielt verändert werden, wodurch sich wiederum ein Benetzungsverhalten an den jeweiligen Oberflächen gezielt einstellen lässt.

Ferner kann als Fertigungsparameter der Abstand zwischen Plattform bzw. letztem polymerisierten Schichtkörper und dem Wannenboden während dem Wiederholen der Schichtherstellungs-schritte verändert werden. Hierdurch können durch die Veränderung einer Höhe der jeweiligen Schichtkörper in vertikaler Richtung weiterhin unterschiedliche Oberflächentopologien ausgebildet werden.

Ferner können als Fertigungsparameter die Abmessungen der von den Mikrospiegeln M der Mikrospiegel-Einheit 6 reflektierten Bildpunkte durch die Abbildungseinheit O während dem Wiederholen der Schichtherstellungsschritte verändert werden. Hierdurch können die Oberflächentopologien weiterhin verändert werden.

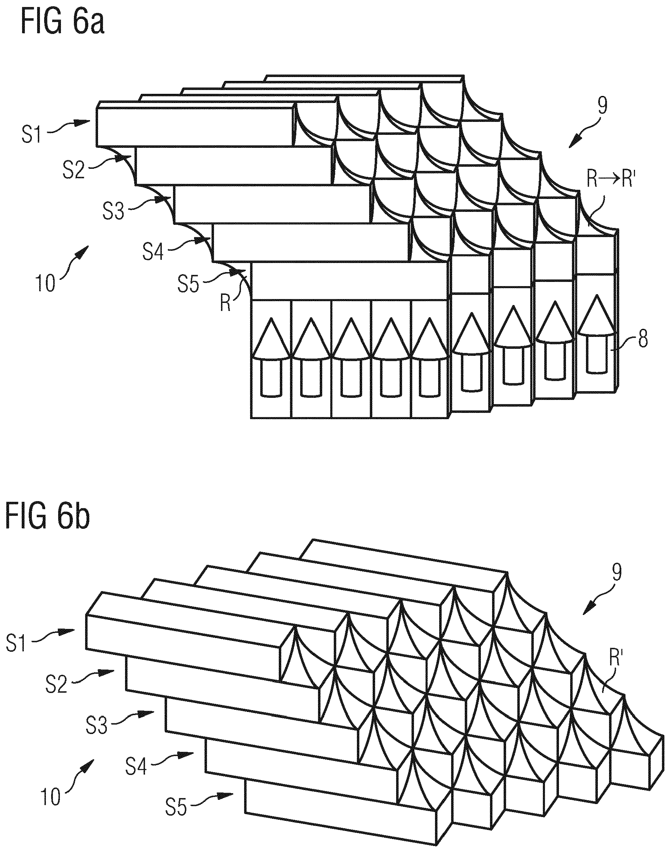

Figuren 6a bis 6c zeigen vereinfachte perspektivische Ansichten zur Veranschaulichung von Verfahrensschritten gemäß einem ersten Ausführungsbeispiel, wobei durch eine Durchbelichtung unterschiedliche Oberflächentopologien gleichzeitig an gegenüberliegenden Oberflächenseiten ausgebildet werden können.

Beispielsweise kann ein 3D-Körper mit der in Figur 6a gezeigten Treppenstruktur durch aufeinanderfolgendes Ausbilden einer Vielzahl von polymerisierten Schichtkörpern S1 bis S5 in üblicher Weise ausgebildet werden. Die Plattform 4 ist in Figur 3a nicht dargestellt und würde sich über dem ersten Schichtkörper S1 befinden. Wie in Figur 6a dargestellt ist, verbleibt bei diesem aufeinanderfolgenden schichtweisen Aufbau jeweils lichtpolymerisierbares Restmaterial R in den Ecken und/oder Kanten einer resultierenden Treppenstruktur. Erfindungsgemäß kann dieser Nebeneffekt dahingehend genutzt werden, dass ein Belichtungsparameter derart verändert (z.B. erhöht) wird, dass beim selektiven Belichten (8) für das Vernetzen eines jeweiligen Schichtkörpers das in den Ecken und/oder Kanten der durchlichtseitigen Treppenstruktur (9) verbleibende lichtpolymerisierbares Restmaterial R durch einen darunterliegenden Schichtkörper (z.B. S5) hindurch belichtet und ebenfalls in lichtpolymerisiertes bzw. vernetztes Restmaterial umgewandelt wird (R -> R'). Das auf der auflichtseitigen Treppenstruktur (10) verbleibende Restmaterial R wird hingegen nicht belichtet (Mikrospiegel M reflektieren das Licht in den Absorber A), weshalb in diesem Bereich keine Polymerisierung bzw. Vernetzung des Restmaterials R stattfindet. Dieses nichtvernetzte Restmaterial kann bei der anschließenden Reinigung teilweise oder vollständig entfernt werden.

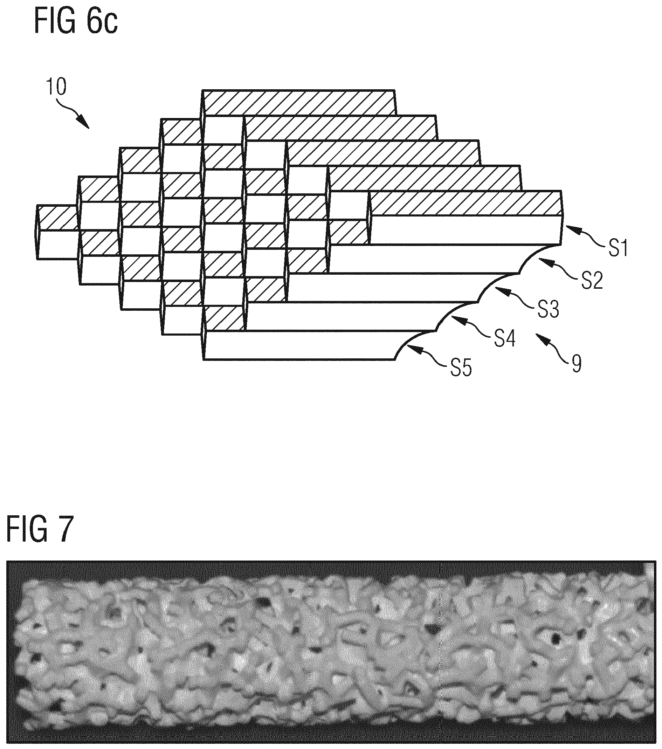

Gemäß Figuren 6b und 6c ergibt sich dadurch für die auflichtseitige Treppenstruktur (10) eine rauere Oberflächentopologie und für die durchlichtseitige Treppenstruktur (9) eine durch das vernetzte Restmaterial R' geglättete Oberflächentopologie (Ecken und/oder Kanten sind aufgefüllt mit polymerisierten Restmaterial R'), wodurch beispielsweise ein unterschiedliches Benetzungsverhalten in diesen lokalen Bereichen realisiert werden kann.

Beispielsweise kann als Belichtungsparameter eine Belichtungsintensität verändert werden, wobei ein Energieeintrag der Strahlung in den jeweils untersten (auszubildenden) Schichtkörper S5 derart erhöht wird, dass auch noch das darüberliegende lichtpolymerisierbare Restmaterial R (durch-) belichtet wird. Somit wird nicht nur der eigentlich auszubildende Schichtkörper S5 sondern auch Restmaterial in einer darüberliegenden Schichtebene polymerisiert bzw. vernetzt, wodurch sich nach einem Reinigungsschritt die dargestellte Glättung der durchlichtseitigen Treppenstruktur 9 ergibt. Da andererseits im Bereich der auflichtseitigen Treppenstruktur 10 keine Belichtung von unten durchgeführt wird, findet auf dieser Seite keine Nachvernetzung des lichtpolymerisierbaren Restmaterials R statt, weshalb nach einem Reinigungsschritt auflichtseitig eine sehr scharfkantige Oberflächentopologie ausgebildet wird. Beispielsweise kann die Belichtungsintensität um einen Faktor 1,5 bis 2,5 gegenüber einer für eine vorbestimmte Schichtdicke normalen Belichtungsintensität erhöht werden.

Alternativ oder zusätzlich kann als Belichtungsparamter eine Belichtungszeit erhöht werden, wobei sich wiederum ein Energieeintrag der Strahlung in den untersten (auszubildenden) Schichtkörper S5 derart erhöht, dass auch noch das darüberliegende Restmaterial R (durch-) belichtet wird. Somit wird nicht nur der eigentlich auszubildende Schichtkörper S5 sondern auch lichtpolymerisierbares Restmaterial in einer darüberliegenden Schicht polymerisiert bzw. vernetzt, wodurch sich die dargestellte Glättung aufgrund des polymerisierten Restmaterials R' auf der durchlichtseitigen Treppenstruktur 9 ergibt. Wiederum wird im Bereich der auflichtseitigen Treppenstruktur 10 keine Belichtung von unten durchgeführt, weshalb auf dieser Seite keine Nachvernetzung des polymerisierbaren Restmaterials R stattfindet und (z.B. bei einer Reinigung das nicht-vernetzte Restmaterial R entfernt und) eine sehr scharfkantige Oberflächentopologie ausgebildet wird. Beispielsweise kann die Belichtungszeit um einen Faktor 1,5 bis 3,5, vorzugsweise Faktor 2, gegenüber einer für eine vorbestimmte Schichtdicke normalen Belichtungszeit verlängert werden.

Weiterhin kann alternativ oder zusätzlich durch Änderung einer Wellenlänge der verwendeten Strahlung ein Energieeintrag erhöht werden, um den vorstehend beschriebenen Effekt zu realisieren.

Obwohl hinsichtlich der Belichtungsparameter regelmäßig von Licht gesprochen wird, kann selbstverständlich jede Art von elektromagnetischer Strahlung (z.B. auch nicht sichtbares Licht, Röntgenstrahlen usw.) für das selektive Belichten verwendet werden.

Gemäß diesem ersten Ausführungsbeispiel können demzufolge durch die gezielte Variation von Belichtungsparametern auf besonders einfache Weise und insbesondere gleichzeitig unterschiedliche Oberflächentopologien an gegenüberliegenden Seiten eines 3D-Körpers bzw. monolithischen Bauteils in einzelnen Oberflächenbereichen ausgebildet werden.

Figur 7 zeigt eine vereinfachte perspektivische Ansicht eines mit dem erfindungsgemäßen Verfahren herstellbaren Gelenkimplantats zur Gewebeneubildung an einem Gelenk.

Gemäß Figur 6 kann zumindest in einem Mantelbereich eine künstliche Trabekularstruktur ausgebildet werden, wobei eine Oberflächentopologie der jeweiligen Trabekel mit dem vorstehend beschriebenen Verfahren gezielt verändert werden kann. Durch die offene und für Körperflüssigkeiten durchlässige Trabekularstruktur des Gelenkimplantats wird grundsätzlich ein schnelles Besiedeln der trabekularen Oberfläche mit Zellen wie Chondroblasten oder Osteoblasten ermöglicht. Ferner kann durch die vorstehend beschriebene gezielte Veränderung der Oberflächentopologie (nicht sichtbar) ein Gelenkimplantat geschaffen werden, wobei in bestimmten Bereichen ein Wachstum eines regenerativen Faserknorpels bis hin zu einem hochwertigen, hyalinen regenerativen Knorpels (Chondroblasten) und in anderen Bereich ein Knochenwachstum (Osteoblasten) begünstigt wird.

Vorzugsweise hat das Gelenkimplantat eine Länge von mindestens 0,6 cm und maximal 1,2 cm für die patellare und Applikation in Kleingelenken wie, z.B. dem Hand- oder Sprunggelenk und mindestens 0,8 cm und maximal 2,2 cm, insbesondere 1,0 cm bis 1,6 cm und weiter bevorzugt 1,25 cm, für die jeweils proximale und distale tibiale und femurale Applikation im Knie- und Hüftgelenk. Dadurch kann ein Optimum an Zugänglichkeit und Einwachsen von mesenchymalen Stammzellen ermöglicht werden. Das Gelenkimplantat gemäß Figur 7 kann ferner einen Durchmesser D von mindestens 2 mm und maximal 6 mm, vorzugsweise 3 mm, aufweisen, wodurch ein Optimum an lateraler, der Synovia (Gelenkspalt) zugewandten Oberfläche zur Ausbildung von Ersatzknorpelgewebe erzielbar ist. Eine Dicke d der künstlichen Trabekularstruktur beträgt vorzugsweise 0,5 bis 2,0 mm, weiter bevorzugt 0,5 bis 1,5 mm.

Durch eine definierte, repetitive, an die natürliche Trabekularstruktur angelehnte Netzstruktur der künstlichen Trabekularstruktur oder eine Kanalstruktur mit entsprechend angepasster Form des Gelenkimplantats und insbesondere in Kombination mit dem vorstehend beschriebenen Verfahren zur Ausbildung von unterschiedlichen Oberflächentopologien in gezielt ausgewählten Bereichen kann ein optimales Einwachsen von körpereigenem Gewebe in das Grenzvolumen zwischen Gelenkimplantat und Vertiefung bzw. Bohrkanal, insbesondere in das Innenvolumen des Gelenkimplantats und oberhalb des zur Synovia zeigenden Endes des Gelenkimplantats ermöglicht werden.

Erfindungsgemäß kann insbesondere ein Gelenkimplantat somit in seinem Deckenbereich und oberen Mantelbereich eine hydrophobe und damit eine eine chondroblastäre Differenzierung begünstigende Oberfläche aufweisen, während der Bodenbereich und der untere Teil des Mantelbereichs eine hydrophile Oberfläche zur Begünstigung einer osteoblastären Differenzierung von mesenchymalen Stammzellen aufweist. Dadurch kann im oberen (der Synovia zugewandten) Bereich des Gelenkimplantats ein Knorpelwachstum und im unteren (dem Knochenmarkraum zugewandten) Bereich des Gelenkimplantats ein Knochenwachstum begünstigt werden.

Alternativ können auch technische Funktionskörper wie z.B. Katalysatoren, Kondensatoren, Membrane usw. als derartige monolithische Bauteile mit lokal unterschiedlichen Oberflächeneigenschaften hergestellt werden. Aufgrund eines selektiv einstellbaren Benetzungsverhaltens können somit technische Funtionskörper (3D-Körper) gezielt und nur für definierte Bereiche z.B. mit elektrisch leitfähigem Material, mit katalytisch wirksamen Material, mit verschleißfestem Material, mit Sensor-Material, mit Kondensator-Material usw. beschichtet werden. Hochkomplexe und sehr kleine Funktionskörper sind damit erstmalig mit relativ geringen Kosten herstellbar.

Es sei darauf hingewiesen, dass jeder Schritt des vorstehend beschriebenen Verfahrens durch Computer-Programm-Befehle implementiert sein kann. Diese Computer-Programm-Befehle können auf einen Computer oder ein anderes programmierbares Gerät geladen werden, um eine Vorrichtung zu erzeugen, wobei die im Computer oder einem anderen programmierbaren Gerät ausgeführten Befehle Mittel zum Implementieren der Funktionsweisen erzeugen, wie sie in den Verfahrensschritten beschrieben sind. Diese Computer-Programm-Befehle können ebenfalls in einem digitalen Speichermedium, wie beispielsweise einer DVD, CD oder Diskette gespeichert sein, die einen Computer oder ein anderes programmierbares Gerät zur Realisierung einer bestimmten Funktionalität anweist. Darüber hinaus können die Computer-Programm-Befehle bzw. der Programm-Code aus beispielsweise einem Telekommunikationsnetzwerk heruntergeladen werden, um Betriebsschritte hervorzurufen, die auf einem Computer oder einem anderen programmierbaren Gerät ausgeführt werden, um einen computer-implementierten Prozess zu erzeugen, der die Durchführung der Verfahrensschritte ermöglicht.

Die Erfindung umfasst daher ferner ein digitales Speichermedium mit elektronisch auslesbaren Steuersignalen, die so mit einem Computer-System einer Vorrichtung zum schichtweisen Aufbau eines monolithischen Bauteils basierend auf einer lithographiebasierten Fertigungstechnik zusammenwirken, dass sie die beschriebenen Verfahrensschritte ausführen.

Ferner bezieht sich die Erfindung auf ein Computer-Programm-Produkt mit auf einem maschinenlesbaren Träger gespeicherten Programm-Code zur Durchführung der beschriebenen Verfahrensschritte, wenn das Programm auf einem Rechner einer Vorrichtung zum schichtweisen Aufbau eines monolithischen Bauteils basierend auf einer lithographiebasierten Fertigungstechnik abläuft. Im Übrigen betrifft die vorliegende Erfindung ein Computer-Programm mit Programm-Code zur Durchführung der vorstehend beschriebenen Verfahrensschritten, wenn das Programm auf einem Computer einer Vorrichtung zum schichtweisen Aufbau eines monolithischen Bauteils basierend auf einer lithographiebasierten Fertigungstechnik abläuft.

Die Erfindung wurde vorstehend anhand von bevorzugten Ausführungsbeispielen beschrieben. Sie ist jedoch nicht darauf beschränkt und umfasst insbesondere auch Kombinationen der vorstehend beschriebenen Ausführungsbeispiele. Insbesondere kann eine Belichtung auch durch Laser anstelle der Mikrospiegel-Einheit erfolgen. Ferner können anstelle des beschriebenen Lithographieverfahrens auch andere additive 3-D-Druckverfahren verwendet werden.

### Bezugszeichenliste

- 1: lichtpolymerisierbares Material
- 2: Wannenboden
- 3: Glättelement
- 4: Plattform
- 5: Belichtungs-Einheit
- 6: Mikrospiegel-Einheit
- 7: monolithisches Bauteil (bzw. Grünkörper)
- 8: selektives Belichten
- 9: durchlichtseitige Treppenstruktur
- 10: auflichtseitige Treppenstruktur
- 11: Bildpunktfeld
- 12: Hubmechanismus
- S1-S5: Schichtkörper
- M: Mikrospiegel
- L: Lichtquelle
- A: Absorber
- P: Bildpunkt (bzw. Pixel)
- O: optische Abbildungseinheit
- E: Entwurfselement
- O_{M}: Oberfläche Entwurfselement
- B: Bauteilelement
- O_{B}: Oberfläche Bauteilelement
- R: lichtpolymerisierbares Restmaterial
- R': lichtpolymerisiertes Restmaterial
- X,Y: horizontale Bauraum-Orientierung (Mikrospiegel-Hauptachsen)
- X_{M}, Y_{M}, Z_{M}: Entwurfselement-Orientierung
- Z: vertikale Bauraum-Orientierung (Lotrechte des Wannenbodens)
- α: Verdrehungswinkel
- βₓ: Verkippungswinkel in x-Richtung
- β_{y}: Verkippungswinkel in y-Richtung
- B₁₁-B₄₄: bei Verkippung hergestellte Bauteile

## Patentansprüche

1. Verfahren zur Herstellung eines monolithischen Bauteils unter Verwendung einer additiven lithographiebasierten Fertigungstechnik, wobei in gezielt ausgewählten Bereichen unterschiedliche Oberflächentopologien erzeugt werden,
**dadurch gekennzeichnet, dass**
die unterschiedlichen Oberflächentopologien zur Realisierung von unterschiedlichen Oberflächeneigenschaften, insbesondere hinsichtlich eines Benetzungsverhaltens, eines Abscheideverhaltens und/oder einer Besiedlung mit Zellen, derart erzeugt werden, dass
das monolithische Bauteil aus einem Entwurf-Bauteil (CAD) mit einer Vielzahl von Entwurf-Elementen (E) erzeugt wird, wobei zumindest eine Entwurfs-Orientierung (X_{M}, Y_{M}, Z_{M}) eines Entwurfelements (E) zu einer Bauraum-Orientierung (X, Y, Z) eines tatsächlichen Bauraums (X, Y, Z) gezielt verändert wird.

2. Verfahren nach Patentanspruch 1, **dadurch gekennzeichnet, dass**
Beim Ausbilden des monolithischen Bauteils unter Verwendung der additiven Fertigungstechnik,
ein lichtpolymerisierbares Material (1) in einer Wanne mit transparentem horizontalen Wannenboden (2) vorgesehen ist, eine Plattform (4) in das lichtpolymerisierbare Material (1) bis zu einem vorbestimmten Abstand zum Wannenboden (2) eingetaucht wird, ein selektives Belichten (8) durch den Wannenboden (2) durchgeführt wird, wodurch ein an der Plattform (4) anhaftender polymerisierter Schichtkörper (Sₓ) mit einer dem Abstand entsprechenden Höhe erzeugt wird,
die Plattform (4) mit dem polymerisierten Schichtkörper (Sₓ) zur Bildung eines nachfolgenden Schichtkörpers (Sₓ₊₁) vertikal verschoben wird,
durch Wiederholen der vorhergehenden Schichtherstellungs-Schritte schichtweise das monolithische Bauteil (7) in der gewünschten Form aufgebaut wird, wobei eine Oberfläche des Bauteils zumindest teilweise eine Treppenstruktur aufweist, und
durch Einstellung von Fertigungsparametern während dem Wiederholen der Schichtherstellungsschritte unterschiedliche Oberflächentopologien zur Realisierung von unterschiedlichen Oberflächeneigenschaften in ausgewählten Bereichen des monolithischen Bauteils (7) erzeugt werden.

3. Verfahren nach Patentanspruch 2,
**dadurch gekennzeichnet, dass** das selektive Belichten (8) mittels einer digitalen Mikrospiegel-Einheit (6) erfolgt, die eine Vielzahl von in Reihen und Spalten angeordneten Mikrospiegeln (M) aufweist.

4. Verfahren nach Patentanspruch 2 oder 3,
**dadurch gekennzeichnet, dass**,
als Fertigungsparameter ein Belichtungsparameter, insbesondere eine Belichtungsintensität und/oder eine Belichtungszeit, beim selektiven Belichten (8) durch den Wannenboden (2) derart eingestellt wird, dass gleichzeitig mit dem zu polymerisierenden Schichtkörper ein in den Kanten und/oder Ecken einer darüber liegenden Treppenstruktur verbleibendes lichtpolymerisierbares Restmaterial (R) polymerisiert wird.

5. Verfahren nach einem der Patentansprüche 2 bis 4,
**dadurch gekennzeichnet, dass** als Fertigungsparameter ein Verdrehungswinkel (α) von horizontalen Mikrospiegel-Hauptachsen (X,Y) gegenüber horizontalen Entwurfselement-Hauptachsen (X_{M}, Y_{M}) während dem Wiederholen der Schichtherstellungsschritte verändert wird.

6. Verfahren nach einem der Patentansprüche 2 bis 5,
**dadurch gekennzeichnet, dass** als Fertigungsparameter ein Verkippungswinkel (β) zwischen einer vertikalen Entwurfselement-Hauptachse (Z_{M}) und einer Lotrechten (Z) des horizontalen Wannenbodens während dem Wiederholen der Schichtherstellungsschritte verändert wird.

7. Verfahren nach einem der Patentansprüche 2 bis 6,
**dadurch gekennzeichnet, dass** als Fertigungsparameter der Abstand zwischen Plattform bzw. letztem polymerisierten Schichtkörper (Sₓ) und dem Wannenboden (2) während dem Wiederholen der Schichtherstellungsschritte verändert wird.

8. Verfahren nach einem der Patentansprüche 3 bis 7,
**dadurch gekennzeichnet, dass** als Fertigungsparameter die Abmessungen der von den Mikrospiegeln (M) der Mikrospiegel-Einheit reflektierten Bildpunkte (P) durch eine Abbildungseinheit (O) während dem Wiederholen der Schichtherstellungsschritte verändert wird.

9. Verfahren nach einem der Patentansprüche 2 bis 8,
**dadurch gekennzeichnet, dass** das lichtpolymerisierbare Material:
einen Werkstoff aus der Klasse der Polymere, insbesondere PA, PEK, PEKK, UHMWPE oder PCL,
einen Werkstoff aus der Klasse der Metalle, insbesondere Ti oder Edelstahl,
einen Werkstoff aus der Klasse der Metall-Legierungen, insbesondere Ti64 oder CoCr,
einen Werkstoff aus der Klasse der Magnesium-Legierungen, insbesondere Mg-Ca, Mg-Zr oder Mg-Zn,
einen Werkstoff aus der Klasse der Keramiken, insbesondere Al₂O₃, ZrO₂, Si₃N₄ oder Ca₃(PO₄)₂,
und/oder
einen Werkstoff aus der Klasse der Gläser beinhaltet.

10. Verfahren nach einem der Patentansprüche 2 bis 9, **dadurch gekennzeichnet, dass** nach der Herstellung des monolithischen Bauteils (7) ein Reinigen, Entbindern und/oder Sintern durchgeführt wird.

11. Verfahren nach einem der Patentansprüche 1 bis 10, **dadurch gekennzeichnet, dass** das monolithische Bauteil (7) ein Gelenkimplantat zur Gewebeneubildung am Gelenk darstellt.

12. Verfahren nach einem der Patentansprüche 1 bis 10, **dadurch gekennzeichnet, dass** das monolithische Bauteil (7) einen technischen Funktionskörper darstellt.

13. Digitales Speichermedium mit elektronisch auslesbaren Steuersignalen, die so mit einem Computersystem einer Vorrichtung zum schichtweisen Aufbau eines monolithischen Bauteils basierend auf einer lithographiebasierten Fertigungstechnik zusammenwirken, dass ein Verfahren nach einem der Patentansprüche 1 bis 10 ausgeführt wird.

14. Computer-Programm-Produkt mit auf einem maschinenlesbaren Träger gespeichertem Programmcode zur Durchführung des Verfahrens nach einem der Patentansprüche 1 bis 10, wenn das Programm auf einem Rechner einer Vorrichtung zum schichtweisen Aufbau eines monolithischen Bauteils basierend auf einer lithographiebasierten Fertigungstechnik abläuft.

15. Computer-Programm mit Programmcode zur Durchführung des Verfahrens nach einem der Patentansprüche 1 bis 10, wenn das Programm auf einem Computer einer Vorrichtung zum schichtweisen Aufbau eines monolithischen Bauteils basierend auf einer lithographiebasierten Fertigungstechnik abläuft.

## Claims

1. Process for manufacturing a monolithic component using an additive lithography-based manufacturing technique, wherein different surface topologies are produced in specifically selected areas, **characterised in that**
the different surface topologies are produced to achieve different surface properties, in particular in terms of wetting behaviour, deposition behaviour and/or colonization with cells, in such a way that
the monolithic component is produced from a component design (CAD) comprising a multiplicity of design elements (E), wherein at least one design orientation (X_{M}, Y_{M}, Z_{M}) of a design element (E) is specifically altered relative to a build space orientation (X, Y, Z) of an actual build space (X, Y, Z).

2. Process according to claim 1, **characterised in that** when forming the monolithic component using additive manufacturing technique,
a photopolymerizable material (1) is provided in a tray having a transparent horizontal tray bottom (2), a platform (4) is immersed in the photopolymerizable material (1) to a predetermined distance from the tray bottom (2), selective irradiation (8) is performed through the tray bottom (2), thereby producing a polymerized layer body (Sₓ) adherent to the platform (4) wich has a height corresponding to the distance, the platform (4) comprising the polymerized layer body (Sₓ) is vertically displaced to form a subsequent layer body (Sₓ₊₁) and by repeating the preceding layer production steps the monolithic component (7) is built up layerwise in the desired shape,
wherein a surface of the component at least partially has a stepped structure, and
by adjusting manufacturing parameters during the repeating of the layer production steps produces different surface topologies for realizing different surface properties in selected regions of the monolithic component (7).

3. Process according to claim 2,
**characterized in that** the selective irradiation (8) is carried out using a digital micromirror unit (6) comprising a multiplicity of micromirrors (M) arranged in rows and columns.

4. Process according to claim 2 or 3,
**characterized in that**, as a manufacturing parameter an irradiation parameter, in particular an irradiation intensity and/or an irradiation time, is in the selective irradiation (8) through the tray bottom (2) adjusted such that simultaneously with the layer body to be polymerized a photopolymerizable residual material (R) remaining in the edges and/or corners of a step structure disposed thereabove is polymerized.

5. Process according to any one of claims 2 to 4,
**characterized in that** as a manufacturing parameter a rotation angle (α) of horizontal micromirror principal axes (X,Y) relative to horizontal design element principal axes (X_{M},Y_{M}) is altered during the repeating of the layer production steps.

6. Process according to any one of claims 2 to 5,
**characterized in that** as a manufacturing parameter a tilting angle (β) between a vertical design element principal axis (Z_{M}) and a perpendicular (Z) to the horizontal tray bottom is altered during the repeating of the layer production steps.

7. Process according to any one of claims 2 to 6,
**characterized in that** as a manufacturing parameter the distance between the platform/the last polymerized layer body (Sₓ) and the tray bottom (2) is altered during the repeating of the layer production steps.

8. Process according to any one of claims 3 to 7,
**characterized in that** as a manufacturing parameter the dimensions of the pixels (P) reflected by the micromirrors (M) of the micromirror unit are altered via an imaging unit (O) during the repeating of the layer production steps.

9. Process according to any one of claims 2 to 8,
**characterized in that** the photopolymerizable material comprises:
a material from the class of polymers, in particular PA, PEK, PEKK, UHMWPE or PCL,
a material from the class of metals, in particular Ti or stainless steel;
a material from the class of metal alloys, in particular Ti64 or CoCr,
a material from the class of magnesium alloys, in particular Mg-Ca, Mg-Zr or Mg-Zn,
a material from the class of ceramics, in particular Al₂O₃, ZrO₂, Si₃N₄ or Ca₃(PO₄)₂, and/or
a material from the class of glasses.

10. Process according to any one of claims 2 to 9,
**characterized in that** the production of the monolithic component (7) is followed by a cleaning, debindering and/or sintering.

11. Process according to any one of claims 1 to 10,
**characterized in that** the monolithic component (7) is a joint implant for tissue regeneration at the joint.

12. Process according to any one of claims 1 to 10,
**characterized in that** the monolithic component (7) is a technical functional body.

13. Digital storage medium containing electronically readable control signals which interact with a computer system of a device for the layer-by-layer construction of a monolithic component based on a lithography-based manufacturing technique, such that a process according to any one of claims 1 to 10 is carried out.

14. Computer program product comprising program code stored on a machine-readable medium for performing the process according to any of Claims 1 to 10, where the program runs on a computer of a device for the layer-by-layer fabrication of a monolithic component based on a lithography-based manufacturing technique.

15. Computer programme comprising programme code for carrying out the method according to any one of claims 1 to 10, where the programme runs on a computer of a device for the layer-by-layer fabrication of a monolithic component based on a lithography-based manufacturing technique.

## Revendications

1. Procédé de fabrication d'un composant monolithique en utilisant une technique de production basée sur la lithographie additive, différentes topologies de surface étant générées dans des zones choisies de manière ciblée, **caractérisé en ce que**
les différentes topologies de surface pour la réalisation de différentes propriétés de surface, en particulier vis-à-vis d'un comportement de mouillage, d'un comportement de dépôt et/ou d'une colonisation par des cellules, sont générées de telle manière que
le composant monolithique est généré à partir d'un composant de conception (CAO) avec une pluralité d'éléments de conception (E), au moins une orientation de conception (X_{M}, Y_{M}, Z_{M}) d'un élément de conception (E) étant modifiée de manière ciblée en une orientation d'espace de construction (X, Y, Z) d'un espace de construction réel (X, Y, Z).

2. Procédé selon la revendication 1, **caractérisé en ce que**
lors de la formation du composant monolithique en utilisant la technique de production additive,
un matériau photopolymérisable (1) est prévu dans une cuve avec un fond de cuve (2) horizontal transparent, une plateforme (4) est immergée dans le matériau photopolymérisable (1) jusqu'à une distance prédéfinie du fond de cuve (2), une exposition sélective (8) est mise en œuvre à travers le fond de cuve (2), ce qui permet de générer un corps stratifié polymérisé (S_{X}) adhérant à la plateforme (4) à une hauteur correspondant à la distance,
la plateforme (4) avec le corps stratifié polymérisé (S_{X})est coulissée verticalement pour former un corps stratifié suivant (S_{X+1}),
en répétant les étapes de fabrication de couches qui précèdent, le composant monolithique (7) est construit couche par couche dans la forme souhaitée, une surface du composant présentant au moins en partie une structure en escalier, et
en réglant des paramètres de production pendant la répétition des étapes de fabrication de couches, différentes topologies de surface peuvent être générées pour réaliser différentes propriétés de surface dans des zones choisies du composant monolithique (7).

3. Procédé selon la revendication 2,
**caractérisé en ce que** l'exposition sélective (8) est effectuée au moyen d'une unité de micromiroirs numériques (6), qui comporte une pluralité de micromiroirs (M) disposés en rangées et en colonnes.

4. Procédé selon la revendication 2 ou 3,
**caractérisé en ce que**
un paramètre d'exposition, en particulier une intensité d'exposition et/ou un temps d'exposition, sont réglés en tant que paramètres de production, lors de l'exposition sélective (8) à travers le fond de cuve (2), de telle manière qu'un matériau résiduel (R) photopolymérisable restant dans les bords et/ou les coins d'une structure en escalier située au-dessus est polymérisé simultanément avec le corps stratifié à polymériser.

5. Procédé selon l'une des revendications 2 à 4,
**caractérisé en ce qu'**un angle de rotation (α) d'axes principaux horizontaux (X, Y) de micromiroirs par rapport à des axes principaux horizontaux (X_{M}, Y_{M}) d'élément de conception est modifié en tant que paramètre de production pendant la répétition des étapes de fabrication de couches.

6. Procédé selon l'une des revendications 2 à 5,
**caractérisé en ce qu'**un angle de basculement (β) entre un axe principal vertical (Z_{M}) d'élément de conception et une droite perpendiculaire (Z) du fond de cuve horizontal est modifié en tant que paramètre de production pendant la répétition des étapes de fabrication de couches.

7. Procédé selon l'une des revendications 2 à 6,
**caractérisé en ce que** la distance entre la plateforme ou le dernier corps stratifié polymérisé (S_{X}) et le fond de cuve (2) est modifiée en tant que paramètre de production pendant la répétition des étapes de fabrication de couches.

8. Procédé selon l'une des revendications 3 à 7,
**caractérisé en ce que** les dimensions des pixels (P) réfléchis par les micromiroirs (M) de l'unité de micromiroirs sont modifiées en tant que paramètres de production par une unité d'imagerie (O) pendant la répétition des étapes de fabrication de couches.

9. Procédé selon l'une des revendications 2 à 8,
**caractérisé en ce que** le matériau photopolymérisable contient :
un matériau de la classe des polymères, en particulier PA, PEK, PEKK, UHMWPE ou PCL,
un matériau de la classe des métaux, en particulier Ti ou acier inoxydable,
un matériau de la classe des alliages métalliques, en particulier Ti64 ou CoCr,
un matériau de la classe des alliages de magnésium, en particulier Mg-Ca, Mg-Zr ou Mg-Zn,
un matériau de la classe des céramiques, en particulier Al₂O₃, ZrO₂, Si₃N₄ ou Ca₃(PO₄)₂,
et/ou
un matériau de la classe des verres.

10. Procédé selon l'une des revendications 2 à 9, **caractérisé en ce qu'**après la fabrication du composant monolithique (7), un nettoyage, un déliantage et/ou un frittage sont mis en œuvre.

11. Procédé selon l'une des revendications 1 à 10, **caractérisé en ce que** le composant monolithique (7) représente un implant articulaire pour la régénération tissulaire sur l'articulation.

12. Procédé selon l'une des revendications 1 à 10, **caractérisé en ce que** le composant monolithique (7) représente un corps fonctionnel technique.

13. Support de stockage numérique avec des signaux de commande lisibles électroniquement, qui coopèrent avec un système informatique d'un dispositif pour la construction couche par couche d'un composant monolithique sur la base d'une technique de production basée sur la lithographie, de telle sorte qu'un procédé selon l'une des revendications 1 à 10 est exécuté.

14. Produit de programme informatique avec un code de programme stocké sur un support lisible par machine pour mettre en œuvre le procédé selon l'une des revendications 1 à 10, lorsque le programme s'exécute sur un calculateur d'un dispositif pour la construction couche par couche d'un composant monolithique sur la base d'une technique de production basée sur la lithographie.

15. Programme d'ordinateur avec un code de programme pour mettre en œuvre le procédé selon l'une des revendications 1 à 10, lorsque le programme s'exécute sur un ordinateur d'un dispositif pour la construction couche par couche d'un composant monolithique sur la base d'une technique de production basée sur la lithographie.
